# EUROPEAN PATENT APPLICATION

(11) **EP 2 405 266 A1**
(43) Date of publication of application: **11.01.2012**
(21) Application number: 10748643.3
(22) Date of filing: 23.02.2010
(51) Int. Cl.: G01N 33/53

(54) **METHOD FOR DIAGNOSING ENDOMETRIOSIS AND DIAGNOSTIC KIT FOR ENDOMETRIOSIS**

(30) Priority: 06.03.2009 JP 2009054082; 28.09.2009 JP 2009223493
(71) Applicant: Mochida Pharmaceutical Co., Ltd., Tokyo 160-8515 (JP)
(72) Inventor: ABE, Yasuhito, Touon-shi Ehime 791-0295 (JP); NABETA, Motowo, Touon-shi Ehime 791-0295 (JP); ITO, Masaharu, Touon-shi Ehime 791-0295 (JP)
(74) Representative: von Kreisler Selting Werner
(86) International application number: PCT/JP2010/052738
(87) International publication number: WO 2010/101047

(57) **Abstract**

It is an object of the present invention to provide: a method for determining endometriosis, in which a blood sample from a subject can be used, and which is capable of determining endometriosis with higher sensitivity and higher precision than those of a conventional method using only a conventional endometriosis marker such as CA125; and a diagnostic kit for carrying out the method of the present invention. The method for determining endometriosis according to the present invention comprises: a step of analyzing an expression of at least one selected from among an anti-syntaxin autoantibody, an anti-PDIK1L autoantibody and an anti-enolase autoantibody; and a step of determining the presence or absence of the onset of endometriosis based on results of said expression analysis.

## Description

### [Technical Field]

The present invention relates to a method for determining the presence or absence of endometriosis, a kit for diagnosing endometriosis, and a method using an anti-syntaxin autoantibody and the like.

### [Background Art]

Endometriosis is a disease, in which the abnormal growth of endometriotic epithelial cells or interstitial cells, and relevant abnormal bleeding and regeneration repeatedly occur during the menstrual cycle in the ovary, the fallopian tube, or other tissues or organs outside of the uterine cavity, such as abdominal cavity and the surface of rectum, thereby damaging the tissues. Endometriosis has characteristics similar to those of malignant tumor, such as the growth of endometriotic tissues in an inappropriate location. However, this is a benign disease. Cause for the onset of endometriosis has not yet been clarified, but menstrual stress has been considered to be one cause. In recent years, the number of patients affected with endometriosis has increased due to delayed marriage, advanced childbearing age, a decrease in the number of childbearing, and the like. In addition, it has been reported that endometriosis causes inflammation, pain, tissue adhesion and the like, and results in infertility at a high rate. Therefore, it is important to detect endometriosis in an early stage and to treat it.

With respect to such endometriosis, it is recommended to conduct ocular inspection via laparoscopy or laparotomy and definite diagnosis via histological diagnosis. However, since these diagnostic methods give pain and burden to patients, in general, more simple vaginal examination, echography, blood test and the like are first carried out. In such a blood test, a marker such as CA125 or CA19-9 is used.

Since such CA125 and CA19-9 have originally been tumor makers used for ovarian cancer and the like, they are not specific to endometriosis. Moreover, the diagnosis using these markers is disadvantageous in that it has a low true positive rate and a high false-positive rate. Hence, it has been desired to develop a novel diagnostic marker for endometriosis, which can be used instead of conventional markers such as CA125.

As diagnostic markers for endometriosis other than conventional markers such as CA125, Patent Literature 1 discloses a gene of NADH dehydrogenase or the like, Patent Literature 2 discloses a histamine-releasing factor, and Patent Literature 3 discloses a protein that is called a tissue factor pathway inhibitor (TFPI-2), for example.

### [Citation List]

### [Patent Literature]

[Patent Literature] JP Patent Publication (Kohyo) No. 2003-531580 A
[Patent Literature] JP Patent Publication (Kokai) No. 2005-49343 A
[Patent Literature] JP Patent Publication (Kohyo) No. 2007-506965 A

### [Summary of Invention]

### [Problems to Be Solved by the Invention]

As stated above, other than CA125 and CA19-9 that have been practically used, various types of diagnostic markers for endometriosis have been studied.

However, the markers described in Patent Literatures 1 and 2 are problematic in that the expression level of the gene in an endometrial cell sample must be measured and collection of the sample causes pain to subjects. In addition, in the case of the marker of Patent Literature 3, the correlation of the marker with endometrial cyst (chocolate cyst) developed in the ovary has been experimentally verified, but the correlation of the marker with endometriosis developed in a location outside of the ovary or moderate endometriosis that has not yet reached chocolate cyst has been unknown.

Thus, it is an object of the present invention to provide: a method for determining endometriosis, in which a blood sample from a subject can be used, and which is capable of determining endometriosis with higher sensitivity and higher precision than those of a conventional method using only a conventional endometriosis marker such as CA125; a diagnostic kit for carrying out the method of the present invention; and a method of using an anti-syntaxin autoantibody and the like for determination of endometriosis and production of the diagnostic kit for endometriosis.

### [Means for Solving Problems]

The present inventors have conducted intensive studies directed towards achieving the aforementioned object and have searched for a component usable as a diagnostic marker for endometriosis from components contained in blood. As a result, the inventors have found that an autoantibody against a specific protein has significant correlation with endometriosis and thus is useful as a marker that is more precise than conventional endometriosis markers, thereby completing the present invention.

The method for determining endometriosis according to the present invention comprises: a step of analyzing an expression of at least one selected from among an anti-syntaxin autoantibody, an anti-PDIK1L autoantibody and an anti-enolase autoantibody in a blood sample; and a step of determining the presence or absence of the onset of endometriosis based on results of said expression analysis.

The diagnostic kit for endometriosis according to the present invention comprises at least one of peptide selected from the group consisting of syntaxin, PDIK1L, enolase, a fragment peptide thereof, a denatured form thereof and a modified form thereof, and a secondary antibody that is able to bind to an autoantibody specifically binding to the above-mentioned peptide and has a marker group.

At least one selected from among an anti-syntaxin autoantibody, an anti-PDIK1L autoantibody and an anti-enolase autoantibody according to the present invention can be used for determination of endometriosis and production of a diagnostic kit for endometriosis.

### [Brief Description of Drawings]

[Figure 1] Figure 1 is a view showing the results obtained by measuring the relative activity of an anti-α-enolase autoantibody in serum samples collected from endometriosis patients, patients with other diseases and healthy subjects according to the method of the present invention.
[Figure 2] Figure 2 is a view showing the results obtained by measuring the relative activity of an anti-PDIK1L autoantibody in serum samples collected from endometriosis patients, patients with other diseases and healthy subjects according to the method of the present invention.
[Figure 3] Figure 3 is a view showing the results obtained by measuring the relative activity of an anti-syntaxin 5 autoantibody in serum samples collected from endometriosis patients, patients with other diseases and healthy subjects according to the method of the present invention.

### [Modes for Carrying Out the Invention]

In the method for determining endometriosis according to the present invention, the expression of at least one selected from among an anti-syntaxin autoantibody, an anti-PDIK1L autoantibody and an anti-enolase autoantibody in the blood sample of a subject is analyzed.

Endometriosis is a disease, in which the abnormal growth of endometriotic epithelial cells or interstitial cells, and relevant abnormal bleeding and regeneration repeatedly occur during the menstrual cycle in the ovary, the fallopian tube, or other tissues or organs outside of the uterine cavity, such as abdominal cavity or the surface of rectum, thereby damaging the tissues. This disease is divided into 4 stages from stage I to stage IV depending on severity. In the present invention, as the severity of endometriosis is increased, the presence of the disease can be determined with higher sensitivity and higher precision. Since CA125 and CA19-9 as endometriosis markers that have been practically used are also used as tumor markers for ovarian cancer and the like, they have high false-positive rates. In contrast, the endometriosis marker according to the present invention has lower false-positive rates in healthy subjects and patients with other diseases than those of the conventional markers, except for abnormal measurement values, in experiments carried out by the present inventors.

The type of a blood sample used in the method of the present invention is not particularly limited. The blood sample used herein includes all of blood itself, plasma and serum. However, since an autoantibody that is a target of expression analysis is contained in serum, plasma or serum is preferably used, and the use of serum is more preferable. Such plasma or serum may be obtained according to an ordinary method, and more specifically, centrifugation or filtration may be applied.

The method of the present invention is considered to be useful for other types of homeotherms, as well as for humans. Accordingly, not only a human-derived blood sample, but also blood samples derived from other types of homeotherms can be used.

A blood sample is used in the method of the present invention. Thus, it is not necessary to excise uterine tissues and the like from patients and to use such tissues as a sample, but blood may be collected. Accordingly, the degree of pain and suffering given to patients can be reduced in the method of the present invention.

Syntaxin is a type of cell membrane-binding protein that forms a SNARE complex associated with the exocytosis of cells.

PDIK1L means serine/threonine-protein kinase. It shows homology of 69% with CLIK1 that is also kinase. The amino acid sequence of PDIK1L is described in LINGCHEN GUO, et. al., Journal of Genetics, Vol. 82, Nos.1 & 2, pp. 27-32 (2003).

### (This publication is incorporated herein by reference.)

Enolase is an enzyme associated with a glycolysis pathway, and examples of the isozyme thereof include α-enolase localized in the cytoplasm, β-enolase localized in muscle cells, and γ-enolase localized in neurons.

In the present invention, the expression of autoantibodies against the aforementioned proteins in blood samples collected from subjects is analyzed. The reason that the expression levels of autoantibodies against the aforementioned proteins are increased in blood samples collected from endometriosis patients has not yet been elucidated. However, by analyzing the expression of these autoantibodies, it becomes possible to determine the presence or absence of endometriosis more precisely than conventional markers do.

In the method for determining endometriosis according to the present invention, in addition to the analysis of the expression of at least one selected from among an anti-syntaxin autoantibody, an anti-PDIK1L autoantibody and an anti-enolase autoantibody, the expression of CA125 or CA19-9 is preferably analyzed. By analyzing the expression of CA125 or CA19-9, as well as the expression analysis of an anti-syntaxin autoantibody and the like, it becomes possible to detect endometriosis with higher sensitivity.

An anti-syntaxin 5 autoantibody is preferable as an anti-syntaxin autoantibody that is a target of expression analysis, and anti-α-enolase autoantibody is preferable as an anti-enolase autoantibody.

In the method for determining endometriosis according to the present invention, the method for analyzing the expression of an autoantibody against syntaxin or the like, CA125, and the like is not particularly limited. For instance, the expression analysis method is not limited to the measurement of the absolute amount or concentration of such an autoantibody in a blood sample, but the relative amount or concentration of such an autoantibody may also be measured. More specifically, the amount, concentration or activity of the aforementioned autoantibody or the like in a blood sample may be measured, for example.

Examples of a specific expression analysis method include immunoassay methods such as ELISA, a method using a protein chip, immunonephelometry, a Western blotting method, RIA, Chemiluminescent Enzyme Immunoassay (CLEIA method), a latex agglutination method, Electrochemiluminescence Immunoassay (ECLIA method), and hemagglutination assay (HA method).

Among various methods, an immunoassay is preferable as a mean for carrying out the above-described expression analysis. The immunoassay has high sensitivity and high precision, and thus, it is able to detect a small change in the concentration of an autoantibody in blood.

When the expression analysis of an autoantibody according to the present invention is carried out by ELISA, the diagnostic kit for endometriosis according to the present invention can preferably be used.

The diagnostic kit of the present invention comprises at least one of peptide selected from the group consisting of syntaxin, PDIK1L, enolase, a fragment peptide thereof, a denatured form thereof and a modified form thereof, and a secondary antibody that is able to bind to an autoantibody specifically binding to the above-mentioned peptide and has a labeling group.

Preferably, the above-described diagnostic kit further comprises an anti-CA125 antibody or an anti-CA19-9 antibody, and a secondary antibody that is able to bind to CA125 or CA19-9 specifically binding to the anti-CA125 antibody or the anti-CA19-9 antibody and has a labeling group. Using such a kit, not only the expression of an anti-syntaxin autoantibody or the like, but also the expression of CA125 or CA19-9 can be analyzed. By combining the analysis results of them, it becomes possible to diagnose endometriosis more precisely.

A fragment peptide of syntaxin or the like is a portion of syntaxin or the like, to which the above-described autoantibody is able to specifically bind. Examples of such a fragment peptide include an epitope itself for an autoantibody against syntaxin or the like, and a peptide containing the epitope. Such epitopes, to which the autoantibody according to the present invention binds, may be determined according to a known method such as an ELI spot assay, an intracellular cytokine staining method, flow cytometry or a T cell growth assay.

To a denatured form of syntaxin or the like, the aforementioned autoantibody is able to specifically bind, and such a denatured form is prepared by subjecting syntaxin or the like to physical treatments such as heating, freezing or ultraviolet ray, or chemical treatments such as a surfactant or a denaturant. For example, such a denatured form may be prepared by treating syntaxin or the like with SDS or DTT.

To a modified form of syntaxin or the like, the aforementioned autoantibody is able to specifically bind, and such a modified form comprises modification of one or more amino acid residues. For example, such a modified form may be prepared by treating syntaxin or the like with glutaraldehyde.

In the present invention, as the above-described peptide, a fragment peptide of syntaxin or the like, which has been subjected to a denaturation treatment, a denatured form of syntaxin or the like, which is also a chemically modified form, and the like may be used.

The above-described peptide may comprise mutation, substitution, deletion and/or addition of one or several amino acid residues, as long as the above-described autoantibody is able to specifically bind thereto.

In the diagnostic kit according to the present invention, the aforementioned peptide, an anti-CA125 antibody and the like may be immobilized on a carrier. The use of such a carrier is advantageous in that such a peptide and the like can be easily separated from the sample. The type of a carrier, on which the aforementioned peptide or the like is to be immobilized, is not particularly limited, as long as it is commonly used in a diagnostic kit. Examples of a material for such a carrier include: resins such as polystyrene, polyamide or polyacrylamide; silica gel; glass; and cotton. Examples of the form of such a carrier include a bead, a film, a thread, a non-woven fabric, and an amorphous gel.

When such a diagnostic kit is used, a blood sample collected from a subject is allowed to act on the above-described peptide or the like. As a result, if the autoantibody against a peptide or the like according to the present invention, CA125, etc. is contained in the blood sample, it specifically binds to the peptide or the like. Subsequently, a protein or the like that has non-specifically bound to the peptide or the like is removed by washing with a buffer or the like, and the above-described secondary antibody is allowed to act on the resultant. The secondary antibody binds to the autoantibody or the like that has bound to the above-described peptide or the like. The secondary antibody is detected by a method that depends on a labeling group used.

As such a labeling group, substances that are commonly used in the field of biochemistry may be used. Examples of such a labeling group include fluorescence emission groups, and enzymes such as peroxidase, alkaline phosphatase or luciferase. It becomes possible to emit light by allowing a detection reagent to act on these enzymes. In a case in which peroxidase is used as a labeling group, for example, the antibody can be detected by the use of a coloring reagent that develops color as a result of oxidation by the peroxidase, a coloring reagent that develops color depending on O₂²⁻ generated from peroxide salts by the action of the peroxidase, and the like.

The concentration or amount of an autoantibody contained in a blood sample is indirectly measured using the degree of color developed, etc. The obtained measurement value may be converted to a relative or absolute concentration, amount, activity, etc. using a calibration curve or the like.

The diagnostic kit of the present invention preferably comprises a normal control sample or an endometriosis control sample. If the present diagnostic kit comprises such a sample, the same experiment is performed on the sample, and the obtained measurement values are compared with the results of the test sample, so that the presence or absence of endometriosis in the subject can be more objectively determined.

In the method using a protein chip, there is used a protein chip, on which the above-described peptide or the like is carried on. When a blood sample is allowed to act on such a protein chip, the autoantibody according to the present invention specifically binds thereto. Subsequently, a non-specifically bound protein or the like is removed by washing with a buffer or the like, and the bound autoantibody may be detected by an ordinary method.

Examples of such a detection method include TOF-MASS such as MALDI-TOF-MASS or SELDI-TOF-MASS. The concentration or amount of an autoantibody can be grasped from a molecular weight peak, other fragment peaks, strengths thereof and the like in the TOF-MASS chart. Alternatively, it is also possible to detect an autoantibody selectively binding to a protein chip, using a secondary antibody that has a labeling group and is capable of binding to each autoantibody, as in the case of ELISA.

In the case of applying immunonephelometry, an antigen, which is specific to the autoantibody according to the present invention in a blood sample, is allowed to bind to the autoantibody, and the blood sample is then irradiated with specific light. Thereafter, turbidity, which depends on the amount of the generated insoluble complex, is measured, so as to obtain the concentration or amount of the autoantibody. Thus, the concentration or amount of the autoantibody is obtained as a relative value that depends on the turbidity. It is also possible to obtain an absolute amount or concentration using the previously prepared calibration curve.

In the case of applying Chemiluminescent Enzyme Immunoassay (CLEIA method), each autoantibody contained in a blood sample is sandwiched between the above-described peptide or the like immobilized on a ferrite particle or the like and a labeled antibody, so as to form an antigen-antibody reaction complex. Subsequently, a detection compound or the like, which depends on a labeling group such as a luminescent substrate, is added, and the luminescent amount may be then measured.
It is to be noted that, in the case of using such ferrite particles, a necessary complex can be purified using a magnet or the like.

In the method of the present invention, after the aforementioned operations, the presence or absence of the onset of endometriosis is determined based on the obtained expression analysis results. That is to say, if endometriosis is developed, or if the symptoms thereof are serious, the concentration or amount of the autoantibody according to the present invention in blood increases. Therefore, based on the expression analysis results of the autoantibody according to the present invention, if the expression level is high, the subject can be determined to have positive results in endometriosis. On the other hand, if the expression level is low, the subject can be determined to have negative results in endometriosis.

In fact, the border between positive and negative results, namely, the cutoff value may be changed, depending on the definitions of endometriosis, the severity thereof, and a method of analyzing the expression of the autoantibody according to the present invention. Accordingly, in the stage at which general standards have not yet been determined, it is necessary that a person who carries out the method of the present invention have previously determined the expression analysis method and the cutoff value by a preliminary experiment and the like, and that the person then perform the measurement.

Furthermore, in the method of the present invention, the expression analysis of CA125 or CA19-9 as a conventional marker may also be carried out, as well as the expression analysis of an anti-syntaxin autoantibody or the like. Thereby, the subject can be determined to be endometriosis-positive, when either the anti-syntaxin autoantibody, etc. or CA125, etc. exceeds its cutoff value. Thus, it becomes possible to detect endometriosis with further higher sensitivity.

### [Examples]

Hereinafter, the present invention will be more specifically described in the following examples. However, these examples are not intended to limit the scope of the present invention. The present invention may be appropriately modified, as long as it is compatible with effect as described above or later. Such modifications are also included in the technical scope of the present invention.

### Example 1 Search for endometriosis marker

### (1) Collection of serum sample

Blood was collected from each of fifty-one endometriosis patients of 20 to 50 years old (mean age: 34.7 ± 7.6 (mean value ± standard deviation)), eighteen healthy female subjects of 22 to 34 years old (mean age: 26.4 ± 3.7), and eighteen patients of 17 to 48 years old (mean age: 32.8 ± 9.3), who were affected with diseases other than endometriosis. The diseases other than endometriosis are as shown in Table 1.

**[Table 1]**

| Disease | Number of patients | Disease | Number of patients |
|---|---|---|---|
| Ovarian cyst | 4 | Endometrial cancer | 1 |
| Uterine leiomyoma | 6 | Septate uterus | 1 |
| Tubal obstruction | 2 | Primary amenorrhea (Turner's syndrome) | 1 |
| Ovarian cancer | 2 | Hydatid mole | 1 |

The collected blood and a procoagulant were placed in a tube, and the tube was then left at rest at room temperature for 1 to 2 hours. Subsequently, a separated serum portion was obtained, and it was then preserved at -20°C, until it was used in tests.

### (2) Preparation of total protein

Human malignant pleural mesothelioma cells (RIKEN Japan, ACC-Meso-1 cells) were procured. Using a Dulbecco's modified Eagle's medium, to which 10% fetal bovine serum and 100 µg/mL aminobenzylpenicillin (10% Dulbecco's modified Eagle's medium solution) had been added, the above-mentioned cells were cultured in s% CO₂ at 37°C. The cells were subcultured in a 10% Dulbecco's modified Eagle's medium twice a week.

The thus cultured human malignant pleural mesothelioma cells were collected, and the cells were then mixed with 4 times its mass of a solubilizing buffer containing 8 M urea, 2% Nonidet P-40 (a surfactant), 2% 2-mercaptoethanol and 10 mM PMSF (a protease inhibitor). The cells were dissolved in the buffer using an ultrasonic homogenizer (Sonifier 250, manufactured by BRANSON). The mixture was centrifuged at 10,000 rpm at 4°C for 12 minutes, and a supernatant containing a protein was then obtained. The obtained supernatant was preserved at -80°C, until it was used in tests.

The temperature of the above-described supernatant was returned to room temperature, and 10 to 50 µg of the supernatant was then mixed with a sample loading buffer consisting of a 0.5% IPG buffer and a solubilizing buffer containing pI3-10 (manufactured by GE Healthcare Biosciences). Subsequently, the prepared sample was loaded onto an isoelectric focusing device (Ettan IPGphor II, manufactured by GE Healthcare Biosciences) equipped with one dimensional electrophoresis gel (pH 3 to 10; 7 cm; Immobiline DryStrip, manufactured by GE Healthcare Biosciences), and electrophoresis was then performed. Thereafter, using 10% acrylamide gel (manufactured by BIO CRAFT Co., Ltd.) as two dimensional electrophoresis gel, two dimensional SDS polyacrylamide gel electrophoresis was performed. At the same time, the concentration of a total protein contained in the cell lysate was measured, separately, using D/C Protein Assay Kit (manufactured by Bio-Rad). After completion of the electrophoresis, the protein contained in the gel was transferred onto a PVDF membrane (manufactured by Millipore). In order to prevent non-specific adsorption, using a 0.1% PBS solution containing 5% skimmed milk and 0.1% Tween20, the aforementioned membrane was blocked at room temperature for 1 hour.

### (3) Determination of endometriosis marker

Among the serum samples obtained in (1) above, three samples from endometriosis patients and four samples from healthy subjects were heated to room temperature, and each sample was then 1000-fold diluted with 5% BSA. The PVDF membrane was incubated at room temperature for 1 hour in the serum-diluted sample and in a solution prepared by dissolving 0.1% Tween20 in a 5% BSA-PBS solution. The resultant PVDF membrane was washed with a 0.1% Tween20-PBS solution three times, and it was then incubated at room temperature for 1 hour in a 5%-BSA-PBS solution of HRP-labeled anti-human IgG (manufactured by Santa Cruz Biotechnology, Inc.). Thereafter, the membrane was washed four times, and an ECL solution (manufactured by GE Healthcare Biosciences) was then allowed to act on the membrane, so that a protein spot specifically binding to an antibody contained in the serum sample was allowed to emit light. The thus emitted light was photographed with a chemiluminescence film (HyperFilm ECL, manufactured by GE Healthcare Biosciences). Moreover, the two dimensional electrophoresis gel was directly stained with Coomassie brilliant blue (CBB), separately.

In order to specify proteins that bound to antibodies specific to endometriosis patients, among antibodies contained in the blood samples, a peptide mass fingerprinting method was carried out using a MALDI TOF-MS system (Voyager DE-PRO, manufactured by Applied Biosystems). CBB-stained two dimensional electrophoresis gel spots, which were specific to endometriosis patients, were excised with a knife. Ammonium bicarbonate was dissolved in 50% acetonitrile to obtain a 12.5 mM solution (pH 8.0), and each spot was then incubated at room temperature for 15 minutes in the obtained solution (400 µL). Thereafter, a supernatant was removed by suction. This step was repeated three times. Subsequently, each spot was incubated at room temperature for 5 minutes in 100% acetonitrile (400 µL), and acetonitrile was then removed by suction. Each spot was dried with an evaporator (VC-36N, manufactured by TAITEC Co., Ltd.), and it was then added to and mixed with 15 µg/mL trypsin in a 25 mM ammonium bicarbonate solution (15 to 30 µL; pH 8.0; manufactured by Promega). The obtained mixture was incubated at 37°C overnight. Subsequently, the spot gel was mixed with an aqueous solution (25 to 50 µL) containing 50% acetonitrile and 5% trifluoroacetic acid, and the obtained mixture was then gently stirred for 60 minutes. This extraction operation was carried out again, and the obtained solutions were then gathered, followed by vacuum concentration. The obtained residue was dissolved in a 0.1% trifluoroacetic acid aqueous solution (10 µL), and ZipTip C18 (manufactured by Millipore) was then applied thereto. Washing with ZipTip and a 0.1% trifluoroacetic acid aqueous solution was repeated five times, and the sample contained in the ZipTip was eluted with 30% acetonitrile (2 µL). Subsequently, the eluted solution (0.5 µL) was loaded onto a sample plate of a MALDI TOF-MS system (Voyager DE-PRO, manufactured by Applied Biosystems). It was mixed with the same volume of matrix solution, acetonitrile solution of 50% α-cyano-4-hydroxycinnamic acid (manufactured by Sigma) and 5% trifluoroacetic acid, and the sample plate was then dried with an evaporator. Using a MALDI TOF-MS system (Voyager DE-PRO, manufactured by Applied Biosystems) and Sequezyme peptide mass standards kit (manufactured by Applied Biosystems), TOF-MS analysis was carried out. Using NCBI nr. 2005.01.06 database in the protein inspector page of http://trypsin.nichd.nih.gov/ucsfhtm13.2/msfit.htm, the obtained TOF-MS data was analyzed by MS-Fit program software program ver. 3.2.1, at a peptide mass error of 150 ppm. During this analysis, taking into consideration the fact that each single peptide may have a single incomplete cleavage, no restrictions were established for the pI range. The MALDI TOF-MS analysis was carried out at least three times for each sample. The specified proteins were as shown in the following table.

**[Table 2]**

| | Isoelectric point | Molecular weight |
|---|---|---|
| α-Enolase | 5.8 | 49478 |
| PDIK1L | 6.4 | 38546 |
| Syntaxin 5 | 9.0 | 34086 |

As shown in the above results, it was found that proteins, to which autoantibodies specifically contained in the serum samples from endometriosis patients bind, are α-enolase, PDIK1L and syntaxin 5.

### Example 2 Measurement of the relative activity value of the autoantibody according to the present invention in each serum sample

### (1) Preparation of recombinant protein

From human malignant pleural mesothelioma cells (RIKEN Japan, ACC-Meso-1 cells), cDNA was prepared. Thereafter, genes each encoding α-enolase, PDIK1L or syntaxin 5, with each of which glutathione S-transferase (GST) was fused, were amplified by a PCR reaction. The amplified genes were each introduced into *Escherichia coli* for transformation, so as to obtain GST-fused α-enolase, GST-fused PDIK1L and GST-fused syntaxin 5.

### (2) Measurement of the relative activity value of each autoantibody in serum sample by ELISA

ELISA was performed using the thus prepared GST-fused proteins, so that the relative activity values of autoantibodies against α-enolase, PDIK1L and syntaxin 5 in the serum samples in the above-described Example 1(1) were measured.

Each GST-fused protein was diluted with a 0.1 M carbonate buffer (pH 9.5), and 100 µL of the diluted solution was then added to wells of an ELISA plate (#3369, manufactured by Corning) to a final concentration of 1 µg/mL. The plate was incubated at 4°C overnight, so that each protein was immobilized thereon. Thereafter, it was washed with a PBS solution of 0.05% Tween20 four times. Then, 1% BSA-PBS (100 µL) was added to each well.

At the start of the assay, 1% BSA-PBS was removed, and each sample (100 µL) that had been 2000-fold diluted with 1% BSA-PBS was then added to wells, followed by incubation at room temperature for 1 hour. Subsequently, HRP-labeled anti-human IgG (100 µL, manufactured by Santa Cruz Biotechnology, Inc.) was added to the resultant, and the obtained mixture was then incubated at room temperature for 1 hour. After completion of a washing operation, 10 mg/mL tetramethylbenzidine dissolved in 0.05 M sodium acetate buffer (pH 5.5) containing 0.01% H₂O₂ (100 µL) was added to the resultant, and the obtained mixture was then incubated at room temperature for 30 minutes. Subsequently, dilute sulfuric acid (50 µL) was added to the reaction solution, so as to terminate the reaction. Using Multiscan ELISA Reader (manufactured by Labosystems), the optical density at 450/620 nm was measured.
The measurement was carried out three times for each sample, and the obtained mean value was used for data analysis.

A standard serum was selected, separately. The serum was 2000-fold diluted with 1% BSA-PBS, and the optical density thereof was then measured in the same manner as described above. Moreover, with respect to 125- to 8000-fold diluted serums and a blank as well, the same measurement was carried out. The activity of each autoantibody in a 2000-fold diluted solution was assumed to be 1000 units/mL, and regarding the relative activity value of each autoantibody and optical density, a calibration curve was then prepared.

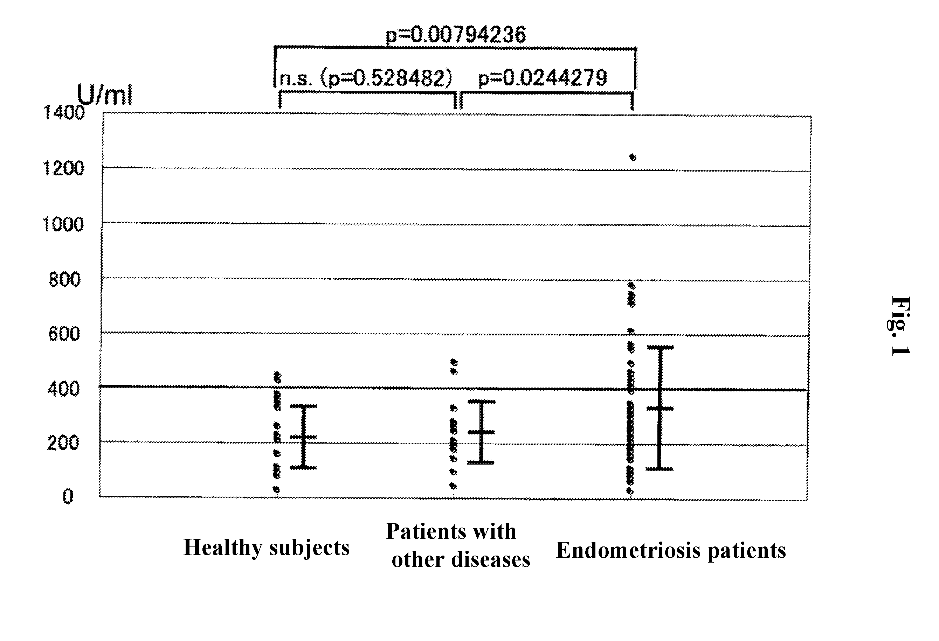

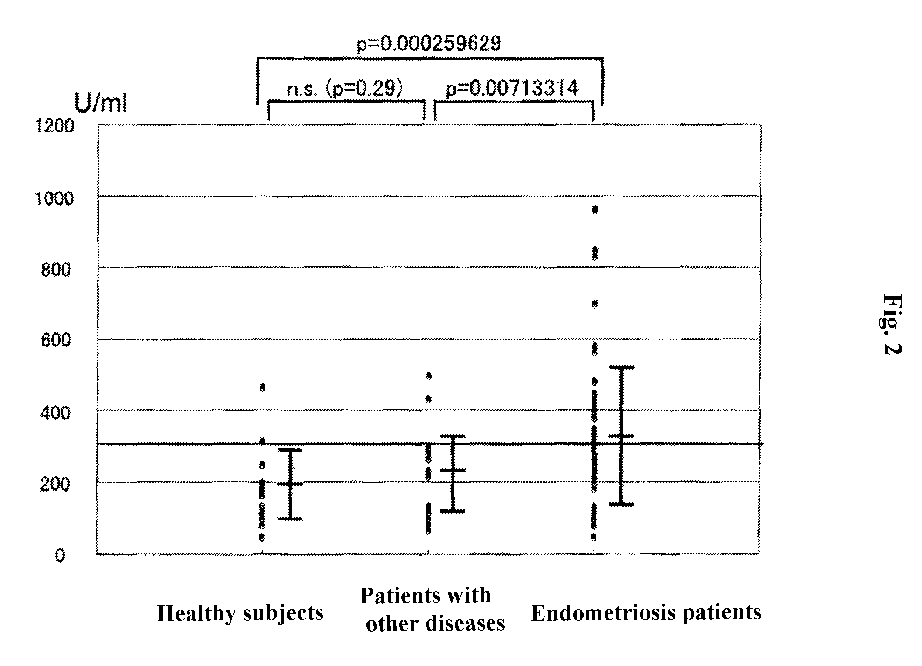

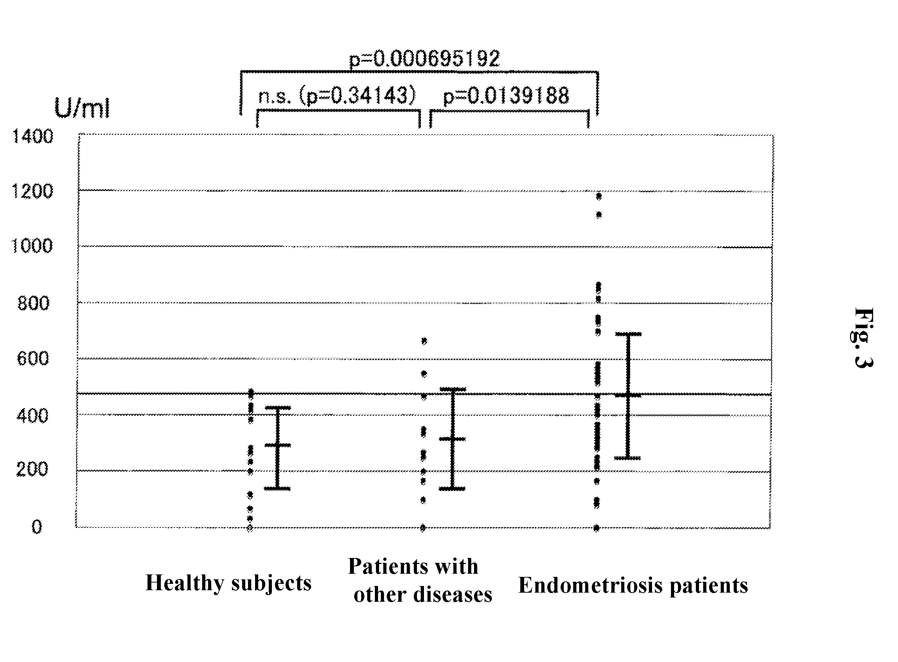

The relative activity values of an anti-α-enolase autoantibody, an anti-PDIK1L autoantibody and an anti-syntaxin 5 autoantibody in each serum sample were obtained from the measurement value of the optical density and the above-described calibration curve. Statistical analysis was carried out by the Welch's t-test, and a ROC (Receiver Operating Characteristic) plot was calculated. The results of the anti-α-enolase autoantibody are shown in Figure 1, the results of the anti-PDIK1L autoantibody are shown in Figure 2, and the results of the anti-syntaxin 5 autoantibody are shown in Figure 3.

As shown in Figure 1, the relative activity value of the anti-α-enolase autoantibody in the serum samples from endometriosis patients is significantly higher than that of healthy subjects at a critical value of p = 0.0079, and it is significantly higher than that of patients with diseases other than endometriosis at a critical value of p = 0.024.

Moreover, as shown in Figure 2, the relative activity value of the anti-PDIK1L autoantibody in the serum samples from endometriosis patients is significantly higher than that of healthy subjects at a critical value of p =0.00026, and it is significantly higher than that of patients with diseases other than endometriosis at a critical value of p = 0.0071.

Furthermore, as shown in Figure 3, the relative activity value of the anti-syntaxin 5 autoantibody in the serum samples from endometriosis patients is significantly higher than that of healthy subjects at a critical value of p =0.00070, and it is significantly higher than that of patients with diseases other than endometriosis at a critical value of p = 0.014.

The relative activity value of CA125 serving as a conventional endometriosis marker in a serum sample was measured by a radioimmunoassay in the Medical Institute of Ehime. As a result, its cutoff value was found to be at a low level (35 units/mL). In particular, even in serum samples from patients with other diseases or healthy subjects, a value higher than the cutoff value was obtained very often, and thus, false-positive cases were observed.

In contrast, in the above-described experiment, the cutoff value of the anti-α-enolase autoantibody was set at 400 units/mL, the cutoff value of the anti-PDIK1L autoantibody was set at 310 units/mL, and the cutoff value of the anti-syntaxin 5 autoantibody was set at 470 units/mL. As shown in Figures 1 to 3, when these cutoff values were adopted, no false-positive cases were observed, except for several cases of serum samples from patients with other diseases and healthy subj ects showing abnormal measurement values.

As described above, it was demonstrated that the method according to the present invention is able to determine the presence or absence of endometriosis more precisely than the conventional method.

### Example 3 Combined use of CA125

Blood was collected from each of the sixty-five endometriosis patients of 20 to 50 years old shown in Table 3, thirty-nine healthy female subjects of 22 to 51 years old (mean age: 35.0 ± 3.7), and thirty-one patients of 17 to 50 years old (mean age: 33.9 ± 9.3), who were affected with diseases other than endometriosis. Thereafter, serum samples were obtained in the same manner as that of the above-described Example 1(1). It is to be noted that the diseases other than endometriosis are as shown in Table 4.

**[Table 3]**

| Stage | Number of people | Age (mean ± standard deviation, range) | Presence or absence of endometrial cyst | Presence or absence of dysmenorrhea |
|---|---|---|---|---|
| I | 13 | 34.8 ± 8.0,22-50 | 0/13 | 9/13 |
| II | 11 | 35.2 ± 9.0,22-48 | 2/11 | 8/11 |
| III | 18 | 36.6 ± 7.8,23-49 | 13/18 | 13/18 |
| IV | 23 | 33.9 ± 7.2,20-50 | 21/23 | 19/23 |
| Total | 65 | 35.0 ± 7.9,20-50 | 36/65 | 49/65 |

**[Table 4]**

| Disease | Number of people | Disease | Number of people |
|---|---|---|---|
| Ovarian cyst | 9 | Cervical cancer | 2 |
| Uterine leiomyoma | 7 | Endometrial hyperplasia | 1 |
| Tubal obstruction | 3 | Septate uterus | 1 |
| Infertility | 2 | Primary amenorrhea (Turner's syndrome) | 1 |
| Ovarian cancer | 2 | Hydatid mole | 1 |
| Endometrial cancer | 2 | | |

The relative activity values (U/mL) of an anti-α-enolase autoantibody and CA125 contained in the serum samples were measured in the same manner as that of the above-described Example 2(2), and statistical analysis was then carried out by the Welch's t-test. The results, together with the results of the below-mentioned Example 4, are shown in Table 7.

Moreover, as in the case of the above-described Example 2(2), when the anti-α-enolase autoantibody was used as an endometriosis marker, the cutoff value in the serum sample was set at 400 U/mL, and when CA125 was used as an endometriosis marker, it was set at 35 U/mL. In a case in which the anti-α-enolase autoantibody exceeded its cutoff value and in a case in which either the anti-α-enolase autoantibody or CA125 exceeded its cutoff value, sensitivity (the number of true positive subjects/the number of endometriosis patients) × 100), specificity ([the number of true negative subjects/(the total number of subjects-the number of endometriosis patients)] × 100), and correct diagnosis rate ([(the number of true positive subj ects + true negative subj ects)/the total number of subj ects] × 100) were calculated. It is to be noted that in the above formulae, the term "true positive" is used to mean endometriosis patients who tested positive, and the term "true negative" is used to mean healthy subjects and patients affected with diseases other than endometriosis, who tested negative. The results, together with the results of the below-mentioned Example 4, are shown in Table 8.

### Example 4 Combined use of CA125

Blood was collected from each of the sixty-nine endometriosis patients of 20 to 51 years old shown in Table 5, forty-four healthy female subjects of 22 to 51 years old (mean age: 34.2 ± 9.3), and thirty-eight patients of 18 to 48 years old (mean age: 34.4 ± 8.2), who were affected with diseases other than endometriosis. Thereafter, serum samples were then obtained in the same manner as that of the above-described Example 1(1). It is to be noted that the diseases other than endometriosis are as shown in Table 6.

**[Table 5]**

| Stage | Number of people | Age (mean ± standard deviation, range) | Presence or absence of endometrial cyst | Presence or absence of dysmenorrhea |
|---|---|---|---|---|
| I | 11 | 37.1 ± 8.7,23-50 | 0/11 | 8/11 |
| II | 10 | 36.1 ± 10.2, 22-49 | 3/10 | 6/10 |
| III | 20 | 34.1 ± 7.2,22-48 | 16/20 | 14/20 |
| IV | 28 | 34.9 ± 7.2,20-51 | 27/28 | 22/28 |
| Total | 69 | 35.2 ± 8.0,20-51 | 46/49 | 50/69 |

**[Table 6]**

| Disease | Number of Number of people | Disease | Number of people |
|---|---|---|---|
| Ovarian cyst | 9 | Tubal obstruction | 2 |
| Uterine leiomyoma | 8 | Cervical cancer | 2 |
| Ovarian cancer | 4 | Septate uterus | 1 |
| Endometrial cancer | 4 | Primary amenorrhea (Turner's syndrome) | 1 |
| Infertility | 3 | Hydatid mole | 1 |
| Adenomyosis | 3 | | |

The relative activity values (U/mL) of a PDIK1L autoantibody, a syntaxin 5 autoantibody and CA125 contained in the serum samples were measured in the same manner as that of the above-described Example 2(2), and statistical analysis was then carried out by the Welch's t-test. The results are shown in Table 7. In terms of CA125, the results of Example 3 are shown in the upper case, and the results of Example 4 are shown in the lower case.

**[Table 7]**

| | Patients with other diseases | Healthy subjects | Endometriosis patients | critical value | |
|---|---|---|---|---|---|
| | | | | Healthy subjects vs. endometriosis patients | Patients with other diseases vs. endometriosis patients |
| α-Enolase | 236.5 ± 119.7 | 223.9 ± 113.8 | 348.6 ± 225.1 | P = 0.00035** | P = 0.00022* |
| PDIK1L | 203.4 ± 129.5 | 155.8 ± 95.2 | 370.2 ± 212.5 | P = 8.4 × 10⁻¹¹** | P = 2.4 × 10⁻⁵** |
| Syntaxin 5 | 287.9 ± 161.8 | 201.5 ± 107.2 | 505.4 ± 381.0 | P = 2.0 × 10⁻⁸** | P = 9.0 × 10⁻⁶** |
| CA125 | 38.5 ± 54.2 | 21.5 ± 5.1 | 43.0 ± 44.3 | P = 0.00028** | P = 0.693 ^{ns} |
| | 281.9 ± 1494.1 | 20.9 ± 10.5 | 54.4 ± 145.7 | P = 0.063^{ns} | P = 0.36^{ns} |

| | | | | | |
|---|---|---|---|---|---|
| **: a case in which there is a significant difference at a critical value of p < 0.001 *: a case in which there is a significant difference at a critical value of p < 0.01 ns: there is no significant difference | | | | | |

As shown in the above results, when the conventional CA125 was used as a marker, endometriosis could not significantly be detected, particularly, with respect to patients affected with diseases other than endometriosis. In contrast, when the marker according to the present invention was used as an indicator, it was demonstrated that endometriosis could significantly be detected at a low critical value, not only with respect to healthy subjects, but also with respect to patients affected with diseases other than endometriosis.

Furthermore, as in the case of the above-described Example 2(2), when the anti-PDIK1L autoantibody was used as an endometriosis marker, the cutoff value in the serum sample was set at 310 U/mL. When the anti-syntaxin 5 autoantibody was used as an endometriosis marker, it was set at 470 U/mL, and when CA125 was used as an endometriosis marker, it was set at 35 U/mL. In a case in which the anti-PDIK1L autoantibody exceeded its cutoff value, in a case in which the anti-syntaxin 5 autoantibody exceeded its cutoff value, in a case in which either the anti-PDIK1L autoantibody or CA125 exceeded its cutoff value, and in a case in which either the anti-syntaxin 5 autoantibody or CA125 exceeded its cutoff value, the sensitivity, the specificity and the correct diagnosis rate were calculated. The results are shown in Table 8.

**[Table 8]**

| | | Sensitivity | Specificity | Correct diagnosis rate |
|---|---|---|---|---|
| α-Enolase | | 36.9% | 91.4% | 65.2% |
| PDIK1L | | 59.4% | 84.1% | 70.2% |
| Syntaxin 5 | | 58.0% | 80.5% | 70.2% |
| CA125+ | α-Enolase | 60.0% | 81.4% | 71.1% |
| | PDIK1L | 73.9% | 72.0% | 69.5% |
| | Syntaxin 5 | 72.5% | 67.1% | 69.5% |

As shown in the above results, when either the marker according to the present invention or the conventional CA125 was used as an indicator, selectivity tended to be decreased when compared with only the marker of the present invention was used as an indicator. Precision was almost equivalent, but sensitivity was significantly improved. These results demonstrated that, when endometriosis needs to be detected in its early stage as precise as possible, the combined use of CA125 with the marker of the present invention enables detection of endometriosis with higher sensitivity.

### [Industrial Applicability]

The endometriosis marker of the present invention enables determination of the presence or absence of endometriosis with higher sensitivity and higher precision than those of conventional endometriosis markers. In addition, since the present invention enables determination of the presence or absence of endometriosis using a blood sample, without requiring a uterine tissue sample, it would cause less pain to subjects. Accordingly, the present invention is able to simply and precisely determine the presence or absence of endometriosis, regarding which the number of patients has increased in recent years, and thus, it is extremely useful.

## Claims

1. A method for determining endometriosis, comprising:
a step of analyzing an expression of at least one selected from among an anti-syntaxin autoantibody, an anti-PDIK1L autoantibody and an anti-enolase autoantibody in a blood sample; and
a step of determining the presence or absence of the onset of endometriosis based on results of said expression analysis.

2. The method for determining endometriosis according to claim 1, wherein an expression of CA125 or CA19-9 is analyzed, in addition to the analysis of the expression of at least one selected from among an anti-syntaxin autoantibody, an anti-PDIK1L autoantibody and an anti-enolase autoantibody.

3. The method for determining endometriosis according to claim 1 or 2, wherein said expression analysis is carried out according to an immunoassay.

4. A diagnostic kit for endometriosis, comprising:
at least one of peptide selected from the group consisting of syntaxin, PDIK1L, enolase, a fragment peptide thereof, a denatured form thereof, and a modified form thereof; and
a secondary antibody that is able to bind to an autoantibody specifically binding to said peptide and has a labeling group.

5. The diagnostic kit for endometriosis according to claim 4, wherein the peptide is immobilized on a carrier.

6. The diagnostic kit for endometriosis according to claim 4 or 5, which further comprises an anti-CA125 antibody or an anti-CA19-9 antibody, and a secondary antibody that is able to bind to CA125 or CA19-9 specifically binding to the anti-CA125 antibody or the anti-CA19-9 antibody and has a labeling group.

7. The diagnostic kit for endometriosis according to claim 6, wherein the anti-CA125 antibody or the anti-CA19-9 antibody is immobilized on a carrier.

8. The diagnostic kit according to any one of claims 4 to 7, which further comprises any one or more of a reagent for detecting the labeling group, a normal control sample and an endometriosis control sample.

9. Use of at least one selected from among an anti-syntaxin autoantibody, an anti-PDIK1L autoantibody and an anti-enolase autoantibody for determination of endometriosis.

10. Use of at least one selected from among an anti-syntaxin autoantibody, an anti-PDIK1L autoantibody and an anti-enolase autoantibody for production of a diagnostic kit for endometriosis.
